# EUROPEAN PATENT APPLICATION

(11) **EP 4 576 824 A1**
(43) Date of publication of application: **25.06.2025**
(21) Application number: 23217961.4
(22) Date of filing: 19.12.2023
(51) Int. Cl.: H04R 25/00

(54) **DETERMINING ACOUSTIC MASS OF EARPIECE WITH MACHINE LEARNING ALGORITHM**

(71) Applicant: Sonova AG, 8712 Stäfa (CH)
(72) Inventor: RAU, Richard, 8006 Zürich (CH); SIGWANZ, Ullrich, 8634 Hombrechtikon (CH); PISLAK, Stefan, 8712 Staefa (CH)
(74) Representative: Sykora & König Patentanwälte PartG mbB

(57) **Abstract**

A method for determining an acoustic mass (26) of an earpiece (14) to be plugged into an ear of a user comprises: receiving user data (22) comprising at least an audiogram of the user; and inputting the user data (22) into a machine learning algorithm (24) and determining the acoustic mass (26) by the machine learning algorithm (24).

## Description

### FIELD OF THE INVENTION

The invention relates to a method, computer program and computer-readable medium for determining an acoustic mass of an earpiece to be plugged into an ear of a user, as well as to a method, computer program and computer-readable medium for training a machine learning algorithm used for determining the acoustic mass.

### BACKGROUND OF THE INVENTION

Hearing devices are generally small and complex devices. Hearing devices can include a processor, microphone, speaker, memory, housing, and other electronical and mechanical components. Some hearing devices have a so-called dome, which is plugged into the ear of the user. Domes may be open, vented (i.e., may have one or more venting channels) or completely closed. Completely closed domes also may be called power domes. The dome is used for inputting the sound from the hearing device into the ear channel and for filtering environmental sound. In general, the filtering of environmental sound is achieved with the acoustic coupling of the exterior to the interior of the ear channel by the dome.

The optimal acoustic coupling is usually determined depending on the user's hearing loss, experience, desired amplification, own voice perception, etc. The acoustic coupling may be formally given by the acoustic mass or cutoff frequency provided by the dome. Too open or too occluding coupling configurations may result in rejection by the user, for example due to a higher risk of feedback, occlusion effect, performance degradation of coupling dependent features (such as beamforming), and direct sound attenuation.

Usually, the proposition of the best dome is based on a rule-based algorithm, finding a balance between rejection metrics dealing with occlusion effect, risk of feedback, etc. for each dome. Due to the high complexity of such algorithms, it is challenging to find a suitable parametrization for an optimal proposition, which is accepted by the user and hearing care professional. Furthermore, the current algorithms typically only consider a subset of the relevant end consumer characteristics, such as monaurality, which can lead to higher non-acceptance if the hearing loss is very asymmetric.

In WO 2021 138 603 A1, patient information is obtained, including audiological diagnostic data and patient-specific data of a patient. The audiological diagnostic data and the patient-specific data are concatenated into an input vector. Using the input vector and a plurality of feature vectors as inputs for a machine learning trained model, a correlation between the input vector and each of the plurality of feature vectors is determined, the plurality of feature vectors corresponding to a plurality of hearing assistance device models. The plurality of hearing assistance device models is ranked based on respective correlations to the input vector and information corresponding to a highest ranked hearing assistance device model is output.

### DESCRIPTION OF THE INVENTION

It is an objective of the invention to simplify the selecting and/or manufacturing an earpiece for a specific user. A further objective is to improve the accuracy of determining a suited acoustic mass of an earpiece for a specific user.

These objectives are achieved by the subject-matter of the independent claims. Further exemplary embodiments are evident from the dependent claims and the following description.

A first aspect of the invention relates to a method for determining an acoustic mass of an earpiece to be plugged into an ear and/or an ear channel of a user. In general, the acoustic mass may be defined by the transfer function of the coupling of the earpiece between an exterior and an interior of the ear with respect to the earpiece. The transfer function may define a phase shift and/or an attenuation of a sound signal at a set of frequencies.

The earpiece may be a dome of a hearing device or hearing aid. The dome may be connected via a hose with the hearing device or hearing aid. The earpiece also may be the housing or a part of the housing of hearing device or hearing aid. A hearing device is adapted for receiving a sound signal, processing the sound signal an outputting the sound to an ear of the user, i.e., the user wearing the hearing device. A hearing aid is a hearing device adapted for processing the sound signal, such that a hearing loss of the user is compensated.

According to an embodiment, the method comprises: receiving user data comprising at least an audiogram of the user; and inputting the user data into a machine learning algorithm, and determining the acoustic mass by the machine learning algorithm.

An audiogram may define hearing abilities of the user at a plurality of frequencies. For example, the audiogram comprises a hearing level of the user at the plurality of frequencies. In general, an audiogram is an indicator of the hearing deficiency and hearing abilities of the user intended for wearing the earpiece. This audiogram and optionally further data are input into a machine learning algorithm, which has been trained to output an acoustic mass, which is optimally provided by the earpiece. As described in more detail below, the machine learning algorithm has been trained with an audiogram, optionally further user data, and acoustic masses of earpieces that have been successfully used by the corresponding users.

With such a machine learning algorithm, a complicated rule-based algorithm can be replaced. The machine learning algorithm easily may be improved, when new user data is available. Furthermore, the output of the machine learning algorithm, i.e., the acoustic mass, may facilitate the fitting of a hearing device, the selection of a suitable dome, the manufacturing of custom earpieces, the manufacturing of individual hearing protectors etc. For hearing protection it also is possible that the machine learning algorithm outputs an optimal attenuation profile. This optimal attenuation profile may be compared to profiles of filters in a hearing protection portfolio and a most suitable filter may be chosen, i.e. a filter with a profile that is least different from the optimal attenuation profile output by the machine learning algorithm.

According to an embodiment, the audiogram comprises at least one of: an air conductance audiogram; a bone conductance audiogram; an ipsilateral audiogram of the ear into which the earpiece is plugged; a contralateral audiogram of the opposite ear; an uncomfortable loudness level (for example at a plurality of frequencies). The audiograms for air conductance, bone conductance and/or uncomfortable loudness level may have different levels at different frequencies (usually between 125Hz and 10.000Hz). In general, the audiogram comprises measurement values of hearing abilities of the user at different frequencies. Such measurement values may comprise a lowest hearing level of the user, a speech hearing level of the user, an uncomfortable loudness level, etc.

These measurement values may have been recorded for air conduction, i.e., sound received by the user via air, and/or bone conduction, i.e., sound received via the cranial bone. Differentiating between air conduction and bone conduction may help in choosing the acoustic mass of the earpiece.

The audiogram may comprise data for the ipsilateral ear, i.e., of the ear into which the earpiece is to be plugged and optionally of the opposite ear. Data for the opposite ear may indicate how strong the hearing deficiencies differ between the ears, which also may help in choosing the acoustic mass of the earpiece.

According to an embodiment, the earpiece is an earpiece of a hearing aid. This may be a dome to be connected to a behind-the-ear hearing aid or a receiver-in-the-canal hearing aid. The earpiece also may be a housing of an in-the-ear hearing aid.

According to an embodiment, the user data additionally comprise an experience level of the user indicative of an experience of the user with the hearing aid. The experience level may comprise the number of years, the user is wearing a hearing device. The experience level may differentiate between a first-time user and a long-term user. For a new user, an acoustic coupling related to a more natural sound setting may be beneficial, i.e., the acoustic mass may be determined lower.

According to an embodiment, the user data additionally comprise a fitting formula for the hearing aid. Such a fitting formula may be a formula translating an audiogram into fitting parameters of a hearing device. Such parameters may comprise frequencies specific and/or input level specific amplification/gain settings. There are standardized and/or widely used fitting formulas, such as NAL-NL2 (National Acoustic Laboratories Non-Linear 2), DSL (Desired Sensation Level), Phonak Digital (Adaptive Phonak Digital, APD), etc. With the knowledge of the fitting formula, the accuracy of the determination of the acoustic mass may be improved.

According to an embodiment, the acoustic mass is an acoustic vent mass, which is directly proportional to the length of a vent and inversely proportional to a cross-sectional area of the vent. As already described, in general, the acoustic mass may comprise indicators for the acoustic coupling behavior of the earpiece. The acoustic mass may be encoded as a geometric shape of a vent, i.e., a channel between an exterior and interior of the earpiece.

According to an embodiment, the machine learning algorithm is an artificial neuronal network, a Gaussian process, a polynomial regression and/or a regression tree.

In particular, artificial neuronal networks, in particular with a plurality of layers, are well suited for modelling complex functions, such as the acoustic mass in dependence of the audiogram and optionally further user data.

The artificial neuronal network may be a multi-layer perceptron (MLP). An MLP is suitable for regression prediction problems, where a real-valued quantity is predicted given a set of inputs. MLPs are suitable for classification prediction problems, where inputs are assigned a class or label. Furthermore, MLP classifiers may handle various types of data. They may also accommodate multiple output classes, making them suitable for multi-class classification problems. An MLP is a multi-layer feed-forward neural network with one input layer, one or more hidden layers and an output layer. Each layer, except the input layer, processes the information from the previous layer and sends the results to the next layer. Generally, a three-layer neural network may approximate the nonlinear mapping with arbitrary precision.

The MLP may be implemented as a regression prediction problem. The MLP may predicting a continuously numeric value. A classification of the dome type may be done based on the continuously numeric value, for example by selecting the dome based on comparing the continuously numeric value thresholds. The predicted continuous numeric value could be the acoustic mass of various dome types.

According to an embodiment, the earpiece is a dome of a hearing aid. In particular, the selection of a dome is a difficult task, when a new hearing aid is fitted, since the dome substantially influences the perception of sound of the user. An unfavorable selected dome may lower the acceptance and satisfaction of the user substantially.

According to an embodiment, the earpiece is a hearing protector. The determination of the acoustic mass also may be performed for selecting other types of earpieces, such as hearing protectors. A hearing protector may be a device, which passively or actively reduce the level of a sound signal to be provided to the ear of the user, for protecting the ear of the user. In this case, the machine learning algorithm also may output an optimal attenuation profile, which is compared to attenuation profiles of different types of hearing protectors for choosing a most suitable hearing protector. For the most suitable hearing protector the attenuation profile may be least different from the optimal attenuation profile output by the machine learning algorithm.

According to an embodiment, the method comprises: selecting a type of earpiece providing the determined acoustic mass. There may be several types of premanufactured earpieces. For example, a set of opened, vented and closed domes. From this set, the earpiece may be selected, where the difference of the acoustic mass of the earpiece and the acoustic mass determined with the method is minimal. In particular, the difference of the log compressed acoustic masses may be used. The selected type may be output by the method, for example on a fitting device.

According to an embodiment, the method comprises: determining geometric dimensions of a vent (such as for example diameter and length) of the earpiece. This already may be done on the side of the manufacturer of the earpiece. These geometric dimensions may be used for manufacturing the earpiece.

According to an embodiment, the method comprises: generating production data for the earpiece. The acoustic mass may be translated into geometric features of the earpiece, such as a geometric shape of a vent of the earpiece. Based on the production data, the earpiece may be manufactured.

A further aspect of the invention relates to a training method for training a machine learning algorithm for determining an acoustic mass of an earpiece to be plugged into an ear of a user. For an artificial neuronal network, such a training method includes determining the weights of the neurons of the artificial neuronal network.

According to an embodiment, the training method comprises: receiving a dataset with records of user data, each record comprising at least an audiogram of a user and an earpiece type used by the user; determining for each record at least one user score of the earpiece type from the user data; generating a filtered dataset by excluding records from the dataset, wherein a record is excluded from the dataset, if the at least one user score is lower than a threshold; determining an acoustic mass for each record from the earpiece type; and training the machine learning algorithm with the filtered dataset.

In general, the input data for training the machine learning algorithm includes the user data to be input into the acoustic mass determination method, optional further user data and an acoustic mass corresponding to the user data.

The acoustic mass is determined from the earpiece type, which is provided in the user data. For example, from a shape of the vent or measurements for each earpiece type, the corresponding acoustic mass may be determined.

In the present training method, a user score is determined from the user data, which indicates, how well the acoustic mass was selected for the scenario encoded in the user data. The user score may indicate how satisfied the user was with the selection of the acoustic mass and/or earpiece and/or how much the acoustic mass improved the compensation of the hearing deficiencies of the user.

The user score is determined from the user data. As an example, the user data may comprise a questionnaire filled out by the user. The results of the questionnaire may be evaluated to determine the user score.

However, also indirect indictors may be used to determine the user score, such as a wearing time of the hearing device or the active usage of sound programs by the user.

According to an embodiment, the user score is or comprises a customer satisfaction score. In this case, the satisfaction of the user with the earpiece is used as indicator, whether the correct acoustic mass has been used.

According to an embodiment, each record of the dataset comprises additionally a wearing time of an earpiece and the user score is a customer satisfaction score determined from the wearing time. An indicator for the satisfaction of the user is the wearing time. As longer the wearing time as higher the satisfaction of the user.

According to an embodiment, the user score is or comprises an intelligibility score. In this case, the improvement of intelligibility of speech or other sounds of a hearing device in combination with the earpiece is used as indicator, whether the correct acoustic mass has been used.

Such an intelligibility score may be determined form specific types of user data without the need for asking the user questions. In general, an intelligibility of speech and/or sounds may be derived from a fitting of a hearing device in combination with an acoustic mass of an earpiece.

According to an embodiment, each record of the dataset comprises additionally a fitting formula of a hearing aid to be used with the earpiece; wherein, for at least one frequency, a desired target gain is determined from the fitting formula; wherein, for the at least one frequency, a feedback threshold limited target gain is determined from the earpiece type; and wherein the intelligibility score is determined from the threshold limited target gain at the at least one frequency.

The target gain of the hearing device in one or more frequencies may be determined from the audiogram and the fitting formula stored in the user data. On the other hand, a feedback threshold limited target gain, i.e., the maximal possible target gain at these one or more frequencies for a specific acoustic mass may be determined from the data for the earpiece type in the user data. Using the feedback threshold limited target gain at the one or more frequencies, an indicator for the intelligibility may be derived for the combination of fitted hearing device and earpiece. Such an indicator may be used as intelligibility score.

According to an embodiment, the training method further comprises: determining an augmentation score for each record from the at least one user score; generating an augmented dataset by at least duplicating the record, when its augmentation score is higher than a threshold. On the one hand, the one or more user scores may be used for filtering out records, which have low user scores, i.e., where the acoustic mass fitted to the scenario in the user data. On the other hand, the one or more user scores may be used for multiplicating records, which have high user scores. To this end, an augmentation score may be determined, which is high, when the one or more user scores are high and which is low, when the one or more user scores are low.

It may be that the user data and/or the acoustic mass of duplicated records are varied, to generate a higher variance in the dataset.

Further aspects of the invention relate to a computer program for determining an acoustic mass of an earpiece to be plugged into an ear of a user with a machine learning algorithm and/or for training the machine learning algorithm, which, when being executed by a processor, is adapted to carry out the steps of the method as described in the above and in the following as well as to a computer-readable medium, in which such a computer program is stored.

For example, the computer program for determining an acoustic mass may be executed in a fitting device. The computer program for determining an acoustic mass and/or for training the machine learning algorithm may be executed in a computing device of a hearing device manufacture. The computer-readable medium may be a memory of the respective device.

In general, a computer-readable medium may be a hard disk, an USB (Universal Serial Bus) storage device, a RAM (Random Access Memory), a ROM (Read Only Memory), an EPROM (Erasable Programmable Read Only Memory) or a FLASH memory. A computer-readable medium may also be a data communication network, e.g., the Internet, which allows downloading a program code. The computer-readable medium may be a non-transitory or transitory medium.

It has to be understood that features of the method as described in the above and in the following may be features of the computer program and the computer-readable medium as described in the above and in the following, and vice versa.

These and other aspects of the invention will be apparent from and elucidated with reference to the embodiments described hereinafter.

### BRIEF DESCRIPTION OF THE DRAWINGS

Below, embodiments of the present invention are described in more detail with reference to the attached drawings.
Fig. 1 shows a hearing device with an earpiece.
Fig. 2 shows different types of domes.
Fig. 3 illustrates a method for determining an acoustic mass according to an embodiment of the invention.
Fig. 4 illustrates a method for training a machine learning algorithm according to an embodiment of the invention.
Fig. 5 shows a diagram with an acoustic mass output by the machine learning algorithm.
Fig. 6 shows a diagram with median wearing times used for determining a customer satisfaction score.
Fig. 7 shows a diagram with a weighting function used for determining a customer satisfaction score.
Fig. 8 shows a diagram with a weighting function used for determining an intelligibility score.

The reference symbols used in the drawings, and their meanings, are listed in summary form in the list of reference symbols. In principle, identical parts are provided with the same reference symbols in the figures.

### DETAILED DESCRIPTION OF EXEMPLARY EMBODIMENTS

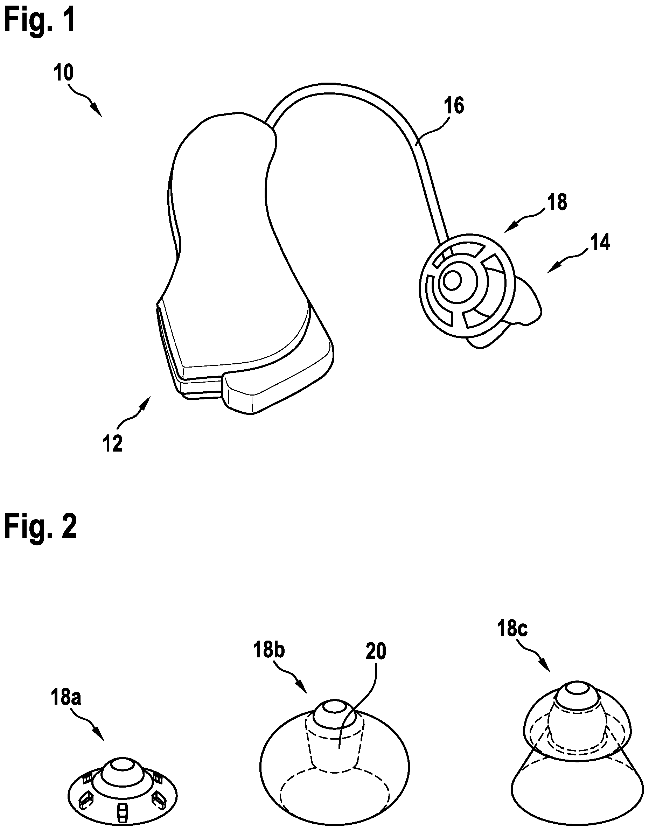

Fig. 1 shows a hearing device 10, in particular a hearing aid, which comprises a part 12 to be worn behind the ear and an earpiece 14 to be plugged into the ear channel of the ear of a user, which user is also called user herein. The part 12 comprises a microphone, sound processor and further components to process the sound signal from the microphone and to compensate hearing deficiencies of a user. The part 12 is connected via a cable 16 with the earpiece 14, which comprises a loudspeaker and a dome 18 to be plugged into the ear channel. More general, the earpiece 14 may be a housing of a hearing device or a hearing protector.

Fig. 2 shows an open dome 18a, a vented dome 18b and a closed dome 18c. The dome 18 has substantially large openings, which allow sound to pass the dome 18a nearly unaltered. The vented dome 18b occludes the ear channel except of a vent 20, which is a small channel interconnecting an inside and an outside with respect to the dome 18b. The closed and/or power dome 18c does not have any openings connecting the inside with the outside and completely occludes the ear channel.

A more open dome 18a has the advantages of less occlusion effect, better wearing comfort and a more natural sound for the user. On the other hand, a more closed dome 18b, 18c has the advantages of better intelligibility, better streaming quality, lower risk of feedback and better sound cleaning. The selection and/or design of a dome 18 or more general an earpiece 14 is a challenging task, which is substantially simplified with the methods proposed herein.

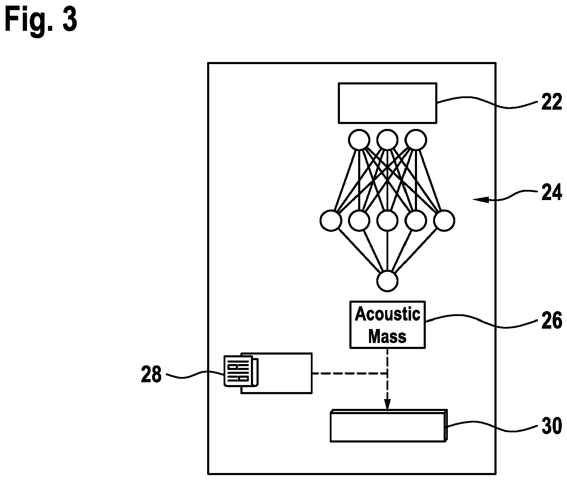

Fig. 3 shows a diagram illustrating a method for determining an acoustic mass 26 of an earpiece 14 to be plugged into an ear of a user and/or user.

The method starts with receiving user data 22. The user data 22 comprises at least an audiogram of the user. Such an audiogram may be recorded at the office of a hearing care specialist. In general, the audiogram may comprise levels of hearing loss at a plurality of frequencies. It also may be that uncomfortable loudness levels are contained in the audiogram at a plurality of frequencies.

More specifically, the audiogram comprises at least one of an air conductance audiogram, a bone conductance audiogram, an ipsilateral audiogram of the ear into which the earpiece is plugged, and/or a contralateral audiogram of the opposite ear. As described above, all these data may improve the accuracy of the method.

Besides the audiogram, the user data 22 additionally may comprise further data relating to the user, such as an experience level of the user indicative of an experience of the user with the hearing aid and/or a fitting formula for the hearing aid 10.

The acoustic mass 26 is determined by a machine learning algorithm 24, which is an artificial neuronal network in the form of a multi-layer perceptron (MLP). The machine learning algorithm 24 has been trained as described herein. The user data 22 is input into the machine learning algorithm 24, which then determines the acoustic mass 26.

The acoustic mass 26 may be one value, but also may be a set of values defining a curve, such as a transfer function of the coupling of the earpiece 14 between an exterior and an interior of the ear with respect to the earpiece. For example, the set of values may comprise phase shifts and/or attenuations of a sound signal at a set of frequencies. An example of an acoustic mass 26 being a single value is an acoustic vent mass, which is directly proportional to the length of a vent 20 and inversely proportional to a cross-sectional area of the vent 20.

Once the acoustic mass 26 has been determined, it may be used for selecting a type 28 of earpiece 14 providing the determined acoustic mass 26.

There may be a list 28 of earpiece types stored in the device performing the method. Each earpiece types 28 may be stored together with its acoustic mass 26, which can be compared with the acoustic mass 26 determined by the machine learning algorithm 24. The method may output the optimal earpiece type and/or optimal dome type, which should be used by the user.

Alternatively or additionally, geometric dimensions 30 of a vent 20 of the earpiece 14 are determined for the acoustic mass 26, which geometric dimensions 30 can be used for manufacturing an earpiece 14.

In particular, the dimensions of a vent 20 (such as diameter and length) may be related to a scalar acoustic mass value, such as an acoustic vent mass. This allows to derive the optimal vent dimension given an earpiece 14 along with the above-described input parameters.

Alternatively or additionally, production data 30 for the earpiece 14 may be generated. For example, this may be done, when custom shells for hearing devices are manufactured.

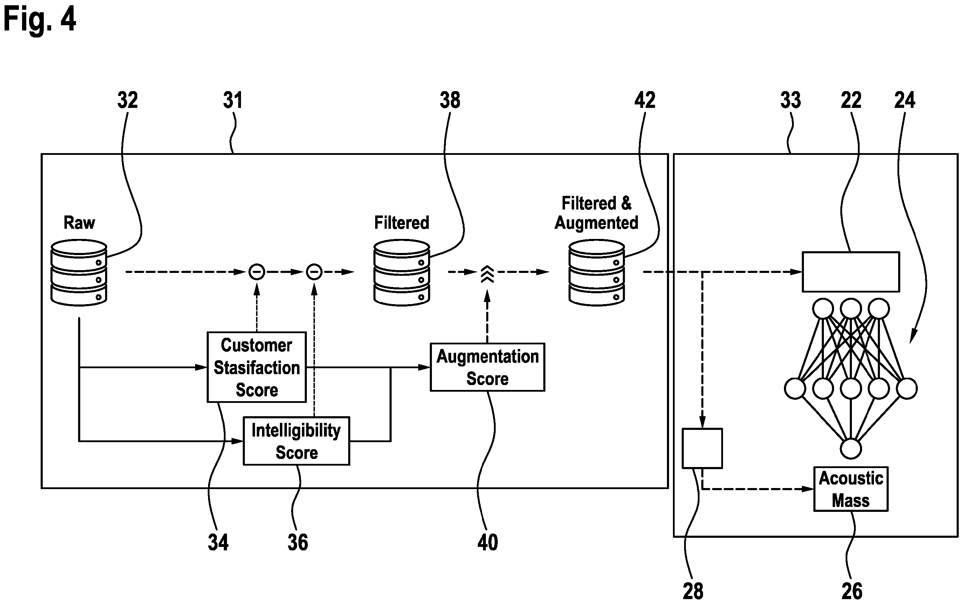

Fig. 4 shows a diagram illustrating the training of the machine learning algorithm 24. The left box 31 illustrates the data preparation, the right box 33 the training with the prepared data.

The starting point is a raw dataset 32, which may include the data of several ten thousand fittings. In particular, every record of the dataset 32 relates to a specific fitting of an earpiece 14 to a user. Every record includes one or more audiograms used for the fitting. Such audiograms may be such as described above, i.e., may include an air conductance audiogram, a bone conductance audiogram, an ipsilateral audiogram of the ear into which the earpiece is plugged and/or a contralateral audiogram of the opposite ear.

Every record furthermore comprises data about the earpiece 14, such as the earpiece type, the geometric size of a vent of the earpiece and/or an acoustic mass of the earpiece 14.

Every record also may include further user data produced during the fitting and/or relating to the fitting, such as questionnaires, wearing times, an age of the user, an experience of the user with hearing devices, etc.

When the fitting related to a hearing device 10 or hearing aid, the record also may comprise hearing device data, such as a fitting formula, parameter of the fitting, etc.

The dataset 32 is received in the beginning of the data preparation. The records at least comprise an audiogram of a user and an earpiece type 28 used by the user.

After that, for each record at least one user score 34, 36 of the earpiece type is determined from the data of the record. As shown, such user scores may be a customer satisfaction score 34 and/or an intelligibility score 36.

With the aid of the user scores 34, 36, a filtered dataset 38 is generated, generating a filtered dataset 38 by excluding records from the dataset 32, wherein a record is excluded from the dataset 32, if the at least one user score 34, 36 is lower than a threshold.

For example, if any of the user scores 34, 36 is below a user score specific threshold, the record is excluded from the dataset 32. For example, the threshold may be 0.5 for the customer satisfaction score 34 and/or 0.9 for the intelligibility score 36. In this case, either the user satisfaction and/or the expected audiological performance is too poor such that the machine learning algorithm 24 should not be trained on the respective record.

It is further possible that an augmentation score 40 is determined for each record of the filtered dataset 38 from the at least one user score 34, 36. The augmentation scores 40 are used for generating an augmented dataset 42. In the augmented dataset 42, a record with an augmentation score 40 higher than a threshold is at least duplicated.

In the case of solely one user score 34, 36, the augmentation score 40 may be the user score and the threshold for duplication is higher than the threshold for filtering.

In the case of at least two user scores 34, 36, the augmentation score 40 may be a weighted average of the at least two scores 34, 36, such as a 50%/50% average. The threshold for the augmentation score 40 may be determined from the filtering thresholds of the user scores.

For example, at an augmentation score 40 of 0.7=0.5*0,5+0,9*0,5, the record may be simply kept, at a value of 1, the record may be used 10 times, for example. This augmentation score 40 scales, how many times the record is duplicated to ensure that the machine learning algorithm 24 is tuned towards high-quality examples.

The data of the duplicated records may be varied. The duplicated records do not have to be completely identical to the original, which, for example, may be achieved by, e.g., adding a small amount of noise to the audiometric data.

For the training, for each record, the user data 22 for training is extracted from each record of filtered dataset 38 or optionally the augmented dataset 42. Furthermore, an acoustic mass 26 for each record is determined from the data relating to the earpiece associated with the record, such as the earpiece type 28. There may be a list of earpiece types 28, wherein a specific acoustic mass 26 has been determined for each earpiece type 28 and has been stored in the list.

The machine learning algorithm 24 is then trained with the filtered dataset 38 and/or the augmented dataset 42, by inputting the user data 22 and the corresponding acoustic mass 26 for each record into the training algorithm.

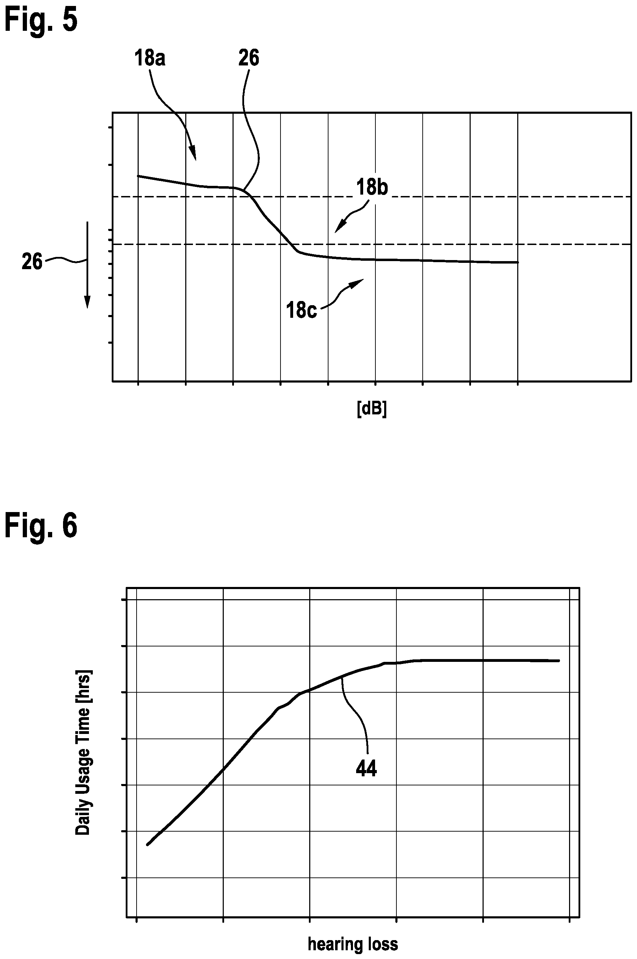

Fig. 5 shows a diagram with a possible output of the machine learning algorithm 24, i.e., the acoustic mass 26 as numerical value, in dependence of a hearing loss. By setting thresholds, a selection of different dome types, such as open domes 18a, vented domes 18b and closed domes 18c, may be performed. For example, with a hearing loss along the fourth vertical line, the predicted optimal acoustic mass 26 would fall into the category of a closed dome 18c.

The benefit in predicting an acoustic mass 26 instead of a dome type lies in the capability to be forward compatible. If, for example, in a future portfolio, the vented dome 18b is replaced by two new vented domes, one closer to the acoustic mass 26 of an open dome 18a, the other closer to the acoustic mass 26 of a closed dome 18c, simply the ranges have to be changed.

Compared to previous proposed algorithms, another novelty of the proposed method is that it is based not only on a monaural hearing loss, but also can take into account a contralateral hearing loss. Additionally, the user experience is considered to capture the preference of first-time users to be more openly fitted. Both parameters were found to improve the prediction accuracy of the machine learning algorithm 24 at testing time.

Returning to Fig. 4, the customer satisfaction score 34 may be determined from the wearing time of the earpiece 14. To this end, each record of the dataset 32 needs to comprise data, from which the wearing time of the earpiece 14 can be determined.

The customer satisfaction score 34 may be derived from an average daily wearing time for each record separately. A normalization may be done based on a hearing loss at different frequencies, such as 500, 1000, 2000, 4000 Hz. Such data may be derived from an audiogram of the user.

Such a normalization is shown in Fig. 6. First, a median wearing time 44 is derived.

This means that users with a milder hearing loss wear the earpiece 14 on average for a shorter period during the day compared to users with a higher hearing loss, which is beneficial to be taken into account when deriving the customer satisfaction score 34.

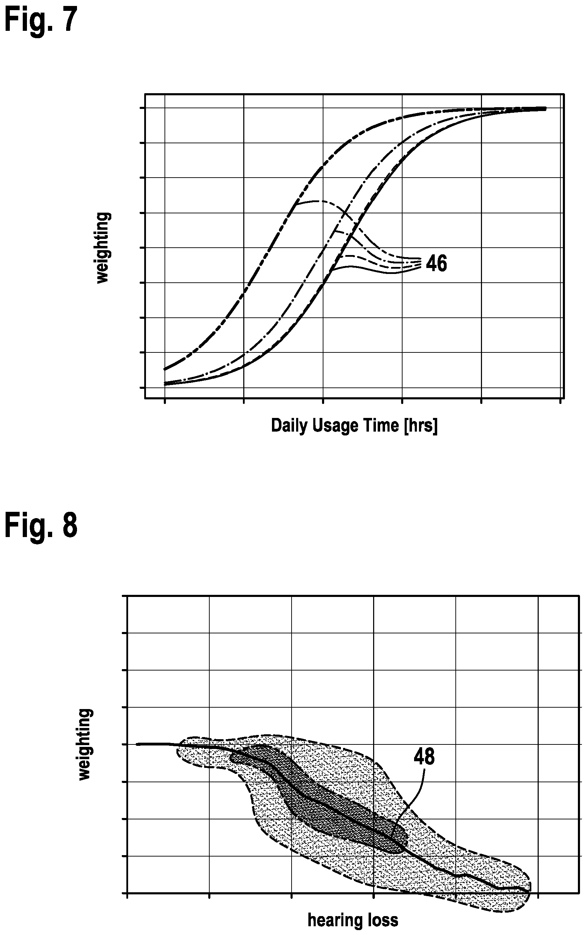

The median wearing time curve 44 is used to derive a weighting function 46 using a sigmoid function for each record. Fig. 7 shows weighting function 46 for different hearing losses. The slope of the function is based on the underlying distribution of the data, i.e., the percentiles.

The customer satisfaction score 34 of a record is then determined by scaling the daily usage time, such as shown in Fig. 6 and multiplying the corresponding weight based on the hearing loss, such as shown in Fig. 7.

The derivation of the customer satisfaction score 34 is described here in a rule-based way. Another approach is to learn data likelihood given a hearing loss profile and wearing time. Such an extension would allow to capture more complex hearing loss dependencies on the wearing time, which may be lost here by computing only the average over four frequencies.

Returning again to Fig. 4, the intelligibility score 36 may be determined based on parameters of a hearing loss of the user and on a fitting formula of a hearing device 10, which is used together with the earpiece 14. In this case, each record of the dataset 32 comprises additionally a fitting formula of the hearing aid 10 to be used with the earpiece 14.

The intelligibility score 36 is derived by the following steps for each record:
Step 1: Computation of the desired target gain based on the fitting formula for 50dB and 65dB speech. In general, for at least one frequency, a desired target gain is determined from the fitting formula.
Step 2: Computation of the estimated feedback threshold for the selected earpiece 14 and derivation of a feedback threshold limited target gain achievable with the earpiece type provided in the record.
Step 3: Computation of an expected intelligibility value with an established intelligibility metric (such as the speech intelligibility index or the derivation of the percentage of correct IEEE sentences) from the feedback threshold limited target gain. This again may be done for at least one frequency and in particular, for both speech input levels at 50dB and 65dB.
Step 4: Derivation of the intelligibility score 36 based on a normalization method. In particular, the intelligibility value of step 3 above is normalized with a weight function. Thus, the intelligibility score 36 is determined from the feedback threshold limited target gain at the at least one frequency.

A weighting function 48 for the intelligibility score 36 is shown in Fig. 8. The weighting function 48 is determined from the intelligibility values of all records determined in step 3. The normalization is based on bucketizing by the hearing loss at different frequencies (which hearing loss is depicted to the right in the diagram).

The intelligibility values in dependence of the hearing loss are shown in Fig. 8 as point clouds. The weighting function 48 is the median of these intelligibility values.

The derivation of the intelligibility score 36 is outlined here in a rule-based way. Another approach is to learn data likelihood given the full audiometric data. This would allow to capture more complex dependencies on the intelligibility, which may be lost here by computing only the average over four AC frequencies.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. A single processor or controller or other unit may fulfill the functions of several items recited in the claims. The mere fact that certain measures are recited in mutually different dependent claims does not indicate that a combination of these measures cannot be used to advantage. Any reference signs in the claims should not be construed as limiting the scope.

### LIST OF REFERENCE SYMBOLS

- 10: hearing device
- 12: behind-the-ear part
- 14: earpiece
- 16: cable
- 18: dome
- 18a: open dome
- 18b: vented dome
- 18c: closed dome
- 20: vent
- 22: user data
- 24: machine learning algorithm
- 26: acoustic mass
- 28: list of earpiece types
- 30: geometric dimensions, production data
- 32: raw dataset
- 34: user score, customer satisfaction score
- 36: user score, intelligibility score
- 38: filtered dataset
- 40: augmentation score
- 42: augmented dataset
- 44: median wearing time
- 46: weighting function
- 48: weighting function

## Claims

1. A method for determining an acoustic mass (26) of an earpiece (14) to be plugged into an ear of a user, the method comprising:
receiving user data (22) comprising at least an audiogram of the user;
inputting the user data (22) into a machine learning algorithm (24) and determining the acoustic mass (26) by the machine learning algorithm (24).

2. The method of claim 1,
wherein the audiogram comprises at least one of:
an air conductance audiogram;
a bone conductance audiogram;
an ipsilateral audiogram of the ear into which the earpiece is plugged;
a contralateral audiogram of the opposite ear;
an uncomfortable loudness level (at a plurality of frequencies).

3. The method of claim 1 or 2,
wherein the earpiece (14) is an earpiece of a hearing aid (10);
wherein the user data (22) additionally comprise at least one of:
an experience level of the user indicative of an experience of the user with the hearing aid;
a fitting formula for the hearing aid (10).

4. The method of one of the previous claims,
wherein the acoustic mass (26) is an acoustic vent mass, which is directly proportional to the length of a vent (20) and inversely proportional to a cross-sectional area of the vent (20).

5. The method of one of the previous claims,
wherein the machine learning algorithm (24) is an artificial neuronal network, a Gaussian process, a polynomial regression and/or a regression tree.

6. The method of one of the preceding claims,
wherein the earpiece (14) is a dome (18) of a hearing aid (10); and/or
wherein the earpiece (14) is a hearing protector.

7. The method of one of the preceding claims, further comprising:
selecting a type (28) of earpiece providing the determined acoustic mass (26).

8. The method of one of the preceding claims, further comprising:
determining geometric dimensions of a vent (20) of the earpiece (14).

9. The method of one of the preceding claims, further comprising:
generating production data for the earpiece (14).

10. A training method for training a machine learning algorithm (24) for determining an acoustic mass (26) of an earpiece (14) to be plugged into an ear of a user, the training method comprising:
receiving a dataset (32) with records of user data (22), each record comprising at least an audiogram of a user and an earpiece type (28) used by the user;
determining for each record at least one user score (34, 36) of the earpiece type from the user data (22);
generating a filtered dataset (38) by excluding records from the dataset (32), wherein a record is excluded from the dataset (32), if the at least one user score (34, 36) is lower than a threshold;
determining an acoustic mass (26) for each record from the earpiece type (28);
training the machine learning algorithm (24) with the filtered dataset (38).

11. The training method of claim 10,
wherein each record of the dataset (32) comprises additionally a wearing time of an earpiece (14);
wherein the user score is a customer satisfaction score (34) determined from the wearing time.

12. The training method of claim 10 or 11,
wherein the user score is an intelligibility score (36);
wherein each record of the dataset (32) comprises additionally a fitting formula of a hearing aid (10) to be used with the earpiece (14);
wherein, for at least one frequency, a desired target gain is determined from the fitting formula;
wherein, for the at least one frequency, a feedback threshold limited target gain is determined from the earpiece type;
wherein the intelligibility score (36) is determined from the feedback threshold limited target gain at the at least one frequency.

13. The training method of one of claims 10 to 12, further comprising:
determining an augmentation score (40) for each record from the at least one user score (34, 36);
generating an augmented dataset (42) by at least duplicating the record, when its augmentation score (40) is higher than a threshold.

14. A computer program for determining an acoustic mass (26) of an earpiece (14) to be plugged into an ear of a user with a machine learning algorithm (24) and/or for training the machine learning algorithm (24), which, when being executed by a processor, is adapted to carry out the steps of the method of one of the previous claims.

15. A computer-readable medium, in which a computer program according to claim 14 is stored.
